Europäisches Patentamt

European Patent Office

Office européen des brevets

(19)

(11) Publication number: **0 185 658**
**B1**

## EUROPEAN PATENT SPECIFICATION

(12)

(45) Date of publication of patent specification: **25.05.88**

(51) Int. Cl.⁴: **A 61 M 1/00**

(21) Application number: **84902956.6**

(22) Date of filing: **11.07.84**

(86) International application number:
**PCT/SE84/00258**

(87) International publication number:
**WO 86/00534 30.01.86 Gazette 86/03**

(54) A SYSTEM FOR THE FLUSHING OF THE CAPSULE OF THE KNEE JOINT.

(43) Date of publication of application:
**02.07.86 Bulletin 86/27**

(45) Publication of the grant of the patent:
**25.05.88 Bulletin 88/21**

(84) Designated Contracting States:
**AT CH DE FR GB LI LU NL**

(56) References cited:
**CH-A- 641 964**
**SE-A-83 027 847**
**US-A-3 860 000**
**US-A-4 117 843**

(73) Proprietor: **GAMBRO AB**
**Post Box 10101**
**S-220 10 Lund (SE)**

(72) Inventor: **HAKANSON, Bo, Hakan**
**Astrakanvägen 6**
**S-223 56 Lund (SE)**
Inventor: **ANDERSSON, Per-Erik**
**Gästgränden 6**
**S-222 51 Lund (SE)**

(74) Representative: **Boberg, Nils Gunnar Erik**
**Gambro AB Patent Department Box 10101**
**S-220 10 Lund (SE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 185 658 B1

## Description

### Technical Field

The present invention relates to an arthroscopy system for continuous flushing of the capsule of the knee joint, which is divided into a flexible tube set intended for single use and a peristaltic pump with associated control system intended for multiple use, the flexible tube set comprising a duct connectable to a liquid source with a pump segment for the pumping of the liquid to a leading-in apparatus for the liquid and a draining duct connectable to the capsule, said first mentioned duct, the capsule and said draining duct being intended to be connected in series.

The system in accordance with the invention is intended for the carrying out of arthroscopy, the said apparatus may therefore also be defined as an arthroscope. Such an apparatus is used for inspection, flushing and possibly treatment of knee caps.

### Background Art

At present two systems for arthroscopy are on the market which supply the arthroscope with liquid during the actual examination. The one system comprises a bag suspended on a column approx. 1.5 m above the patient. From the bag, a flexible tube then leads down to the arthroscope, and the inflow into the same is regulated with the help of a valve on the arthroscope itself. The liquid is drained from the knee with the help of a discharge cannula and a flexible tube. On this flexible tube, a clip is provided by means of which the flow can be opened and shut. The weakness of the system is that in case of the knee having expanded, the pressure from inside the knee will also act upon the flow, which means that the latter has to be adjusted all the time during treatment, according to the expansion of the knee.

In accordance with another system, liquid is supplied to the arthroscope instead with the help of an arthroscopy pump, the rate of which can be controlled by means of a pedal. In this manner, the flow can be controlled without any need for using the hands, which are required partly to manoeuver the arthroscope, partly to control the other operational instruments which are introduced into the knee cap.

A further advantage of using a pump, is that the pump can overcome the internal pressure which is present in the knee. Consequently, it is possible to keep the flow through the knee relatively constant. The flow made use of there ranges from drop rhythm up to 300—400 ml per min. If, for example, an electric "shaver" is used for taking out material, flows right up to 700 ml/min may be required. Owing to the height of the ceiling in normal operating theatres, such flows can be obtained only with the help of pumps, and not with the help of the system described first, which normally gives only a pressure of approx. 150 cm water gauge.

If a pump is used as a driving source, appreciably higher pressures can be obtained. However, there is a risk involved here that the physician, through maladjustment of the pump or careless handling of the same, may burst the capsule of the knee joint. As a result of this, certain physicians, who previously never had to do with arthroscopy, feel apprehensive at the prospect of using the system. It also means that in countries such as eg the USA, where the physician very easily can be sued for wrong treatment, it would be desirable to provide further protection on the equipment so as to make it possible, for example, to monitor the maximum pressure which the pump can attain.

As further background art reference may be made to US—A—3 860 000, US—A—4 117 843 and CH—A—641 964. Especially US—A—3 860 000 describes a system including some details similar to the details used in the system according to the present invention. The system is, however, intended for the feeding, aspirating and irrigating of the stomach of a patient and the problems intended to be solved are therefore quite different compared with the problems intended to be solved by the present invention.

### Disclosure of Invention

To prevent the abovementioned risks, a system of the aforementioned type has been developed in accordance with the invention, which is characterized by a pressure gauge (also referred to hereinafter as a pressure pick-up) connectable to the duct between the pump and the capsule, which is adapted to control a draining valve provided in the draining duct for the control of the pressure in the capsule.

The said pressure gauge can be adapted so as to open the valve in the draining duct when a certain, preferably adjustable, pressure is attained in the capsule. Should the pressure in the capsule increase further, in spite of the opening of the valve, the pressure gauge may be adapted further to stop the rise in pressure by means of adjusting the pump or wholly stopping the pump when a certain, preferably adjustable, maximum pressure is attained in the body cavity. It is possible thus to counteract undesirable pressure increases and, moreover, to prevent completely that a certain maximum value is exceeded.

Means for the joint control of the said two adjustable pressures are suitably arranged so that the valve in the draining duct always opens when the pressure in the capsule attains a certain fraction of the said maximum pressure.

To give the physican even greater freedom of action, the valve in the draining duct may also be controllable by hand.

The system in accordance with the invention is divided appropriately into a flexible tube set intended for single use, comprising a pump segment and a peristaltic pump eg a normal hose pump, with associated control system, intended for multiple use. The flexible tube set may have a branch duct connected downstream of the pump segment, this branch duct being capable of being connected to the pressure pick-up via a liquid-tight but gas-permeable filter. In this manner, the pressure pick-up intended for multiple use is not contaminated. Alternatively, a wholly impermeable and elastic filter may be used for the same purpose.

The said branch duct, furthermore, may comprise a vent valve, which preferably is constituted of a self-closing membrane which can be penetrated by a cannula. In this way, the system can be vented in a simple manner by a conventional injection syringe. Alternatively, a liquid-tight but gas-permeable membrane can be used for this purpose through which the system is continuously vented.

### Brief Description of the Drawings

In the following, the invention will be described further with reference to the enclosed drawings, which, by the way of an example, show a preferred embodiment of the system in accordance with the invention.

Fig. 1 shows schematically a relatively complete system according to the invention in its practical embodiment.

Fig. 2 and 3 show in the form of diagrams how the flow and the pressure respectively can be altered in conjunction with a treatment using the system in accordance with the invention.

### Best Mode of Carrying Out the Invention

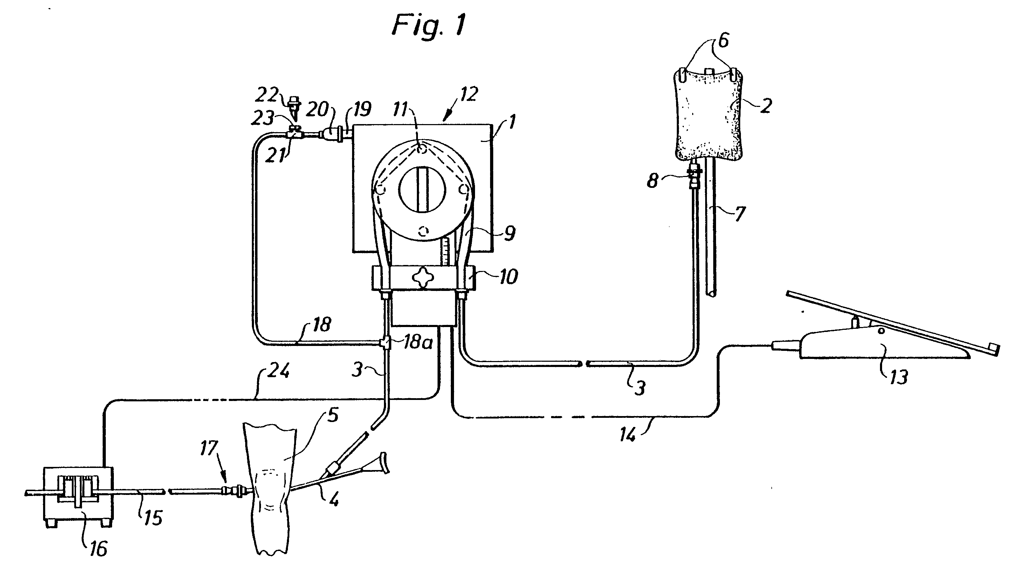

In fig. 1 a system intended for arthroscopy is shown as an example of the system in accordance with the invention. This system comprises a pump 1 for the pumping of liquid from a liquid source 2 via a duct 3 to a leading-in apparatus 4 for the liquid, which in the example consists of an arthroscope for the pumping of the liquid into a knee joint 5. The liquid source 2 is constituted in the example shown of a bag suspended on two hooks 6 in a stand 7. The connection between the duct 3 and the bag 2 is designated 8. In the simplest case, this connection may consist of a simple cannula inserted into the bag. The duct 3 consists of two parts and a pump segment 9 arranged in between which may consist, for example, of silicone rubber, whilst the duct itself consists of a somewhat more rigid material, e.g. PVC. With the help of a clamping device 10 the pump segment 9 is stretched over three of four rolls 11 on a pump rotor 12. By a modification of the clamping device the capacity of the pump can be altered. This can be done with the help of a pedal 13 which is connected to the driving motor of the pump via an electric cable 14.

The system shown further comprises a draining duct 15, connectable to the knee, with a valve 16. The connection is made with the help of the cannula 17 shown schematically.

The duct 3 comprises, moreover, a branch duct 18, which is connected to the main duct with the help of a T-piece 18a at a point following the pump segment 9. This branch duct 18 is connectable to a pressure pick-up 19 via a liquid-tight but gas-permeable filter 20. The branch duct further comprises a vent valve 21 which, in the example shown, includes a self-closing membrane 23 which is penetrable by a cannula 22. The pressure pick-up 19 which, in fig. 1 is indicated only by its outer connecting part, is adapted so that, via a cable 24, it controls the valve 16, and hence the pressure in the knee 5. This is done appropriately in that the valve 16 is opened when a certain pressure is attained in the knee 5. A further safety is obtained if the pressure pick-up, moreover, is adapted so as to stop further rise in pressure by adjusting or wholly stopping the pump when a certain maximum pressure in the knee is attained. Such a situation can arise, for example, if the draining duct for some reason, becomes stopped up.

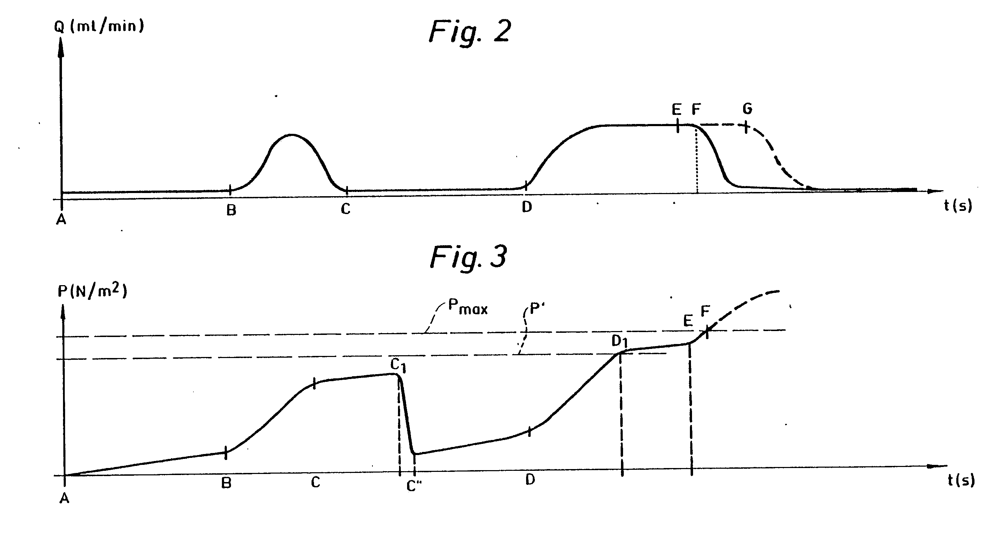

In fig. 2 is shown by way of example how the flow Q can be varied in conjunction with the use of the system in accordance with the invention for arthroscopy. At A a normal flow is initiated which at a time B is increased, for example, for the flushing away of blood for the improvement of visibility. This is done appropriately manually by the physician. At point C, the flow returns to normal, to be increased again at D, for example, for the flushing away of material after an operation. Should a stopping up occur in this connection in the draining duct 15 at E the flow continues wholly or partly unchanged up to a point F. If the stopping up is total, the pump stops altogether, so that the flow drops according to the dotted line. At a partial stoppage, the control is changed over instead from a control of constant flow to a control of constant pressure, the flow possibly diminishing in accordacne with the fully drawn line. The broken line indicates what would happen instead in a system without protective devices in accordance with the present invention. Here the flow would continue unchanged up to the point G, where at best it would be changed over manually by the physician. If this fails to be done, the flow would continue unchanged until a bursting of the capsula of the knee-joint takes place.

In fig. 3 is shown a pressure diagram corresponding to the flow diagram described above. Thus at A a normal flow is initiated the closed valve 16 closed. The pressure P then gently increases up to point B when the flow is increased, for example, for the flushing away of blood or the like. The increased flow brings about a rapid rise in pressure up to point C where the flow returns to normal. The pressure then rises slowly up to point C, where the physician opens valve 16 by hand. At C'' the valve is closed again, whereupon the pressure commences again to increase. This pressure increase is intensified at point D and reaches at point $D_1$, a level P', where valve 16 is opened automatically. The pressure then can be maintained substantially constant, or it increases slowly up to point E where the draining duct is wholly or partially stopped up. This brings about a more rapid rise in pressure up to a level P max at point F, where the pump is changed over from control of constant flow to control of constant pressure. Hence, this pressure level is not exceeded. By contrast, the broken line indicates what might happen in a system without protective arrangements in accordance with the present invention. The pressure would then rise until either the pump is shut off, or switched over by hand or until the capsule of the knee joint has burst.

Naturally the invention is not limited solely to the particulars described above, but can be varied within the scope of the following claims. For example, peristaltic pumps other than that shown may also be used.

## Claims

1. An arthroscopy system for continuous flushing of the capsule (5) of the knee joint, which is divided into a flexible tube set (3, 9, 15, 18) intended for single use and a peristaltic pump (1) with associated control system intended for multiple use, the flexible tube set comprising a duct (3) connectable to a liquid source (2) with a pump segment (9) for the pumping of the liquid to a leading-in apparatus (4) for the liquid and a draining duct (15) connectable to the capsule (5), said first mentioned duct (3), the capsule (5) and said draining duct (15) being intended to be connected in series, characterized by a pressure gauge (19) connectable to the duct (3) between the pump (1) and the capsule (5), which is adapted to control a draining valve (16) provided in the draining duct (15) for the control of the pressure in the capsule (5).

2. A system in accordance with claim 1, characterized in that the said flexible tube set has a branch duct (18) connected downstream of the pump segment (9), this branch duct being connectable to the pressure gauge (19) via a liquid-tight but gas-permeable filter (20) or an elastic, tight membrane.

3. A system in accordance with claim 2, characterized in that the said branch duct (8) comprises further a vent valve (21), which preferably comprises a self-closing membrane (23) which can be penetrated by a cannula (22).

4. A system in accordance with any one of claims 1—3, characterized in that the pressure gauge (19) is adapted to open the valve (16) in the draining duct (15) when a certain, preferably adjustable pressure in the capsule (5) is attained.

5. A system in accordance with any one of claims 1—4, characterized in that the pressure gauge (19) is adapted to stop further rise in pressure through adjustment of the pump (1) or complete stopping of the pump when a certain, preferably adjustable maximum pressure in the capsule (5) is attained.

6. A system in accordance with any one of claims 1—3, characterized in that the valve (16) in the draining duct (15) is also controllable manually.

7. A system in accordance with claim 4 and 5, characterized by means for the ball joint control of the said two adjustable pressures, so that the valve (16) in the draining duct (15) always opens when the pressure in the capsule (5) attains a certain fracture of the said maximum pressure.

## Patentansprüche

1. Arthroskopiesystem zum kontinuierlichen Spülen der Kapsel (5) des Kniegelenkes, welches geteilt ist in eine flexible Schlauchgruppe (3, 9, 15, 18), die für einmalige Verwendung bestimmt ist und eine Schlauchquetschpumpe (1) mit angegliedertem Kontrollsystem, die für mehrmalige Verwendung bestimmt sind, die flexible Schlauchgruppe umfassend eine Rohrleitung (3) verbindbar mit einer Flüssigkeitsquelle (2) mit einem Pumpensegment (9) zum Pumpen der Flüssigkeit zu einer Einführeinrichtung (4) für die Flüssigkeit und einer Drainagerohrleitung (15), verbindbar mit der Kapsel (5), wobei die zuerst genannte Rohrleitung (3), die Kapsel (5) und die Drainagerohrleitung (15) bestimmt sind, um in Serie verbunden zu werden, gekennziechnet durch ein Druckmeßinstrument (19), das mit der Rohrleitung (3) zwischen der Pumpe (1) und der Kapsel (5) verbindbar ist und geeignet ist, ein Drainageventil (16) zu steuern, das in der Drainagerohrleitung (15) vorgesehen ist zum Steuern des Druckes in der Kapsel (5).

2. System nach Anspruch 1, dadurch gekennzeichnet, daß die flexible Schlauchgruppe eine Zweigleitung (18) hat, die stromabwärts des Pumpensegmentes (9) verbunden ist, wobei diese Zweigleitung mit einem Druckmeßinstrument (19) verbindbar ist über einen flüssigkeitsdichten, aber gasdurchlässige Filter (20) oder eine elastiche, dichte Membran.

3. System nach Anspruch 2, dadurch gekennzeichnet, daß die Zweigleitung (18) ferner ein Entlüftungsventil (21) umfaßt, das bevorzugt eine selbstschließende Membran (23) umfaßt, die mittels einer Kanüle (22) durchbohrt werden kann.

4. System nach einem der Ansprüche 1 bis 3, dadurch gekennzeichnet, daß das Druckmeßinstrument (19) so angepaßt ist, daß es das Ventil (16) in der Drainagerohrleitung (15) öffnet, wenn ein gewisser, bevorzugt einstellbarer Druck in der Kapsel (5) erreicht ist.

5. System nach einem der Ansprüche 1 bis 4, dadurch gekennzeichnet, daß das Druckmeßinstrument (19) so angepaßt ist, daß es einen weiteren Anstieg des Druckes durch Einstellen der Pumpe (1) stoppt oder vollständiges Stoppen der Pumpe, sobald ein gewisser, bevorzugt einstellbarer Maximaldruck in der Kapsel (5) erreicht ist.

6. System nach einem der Ansprüche 1 bis 3, dadurch gekennzeichnet, daß das Ventil (16) in der Drainagerohrleitung (15) auch manuell steuerbar ist.

7. System gemäß den Ansprüchen 4 und 5, gekennzeichnet durch Mittel für die Gelenkkontrolle der beiden einstellbaren Drücke, so daß das Ventil (16) in der Drainagerohrleitung (15) immer öffnet, wenn der Druck in der Kapsel (5) einen gewissen Bruchteil des Maximaldruckes erreicht.

**Revendications**

1. Système d'arthroscopie pour le curage continu de la capsule (5) de l'articulation du genou, qui est divisé en un dispositif à tube souple (3, 9, 15, 18), prévu pour un usage unique, et en une pompe péristaltique (1) avec un système de commande associé, prévue pour un usage multiple, le dispositif à tube souple comprenant un conduit (3) connectable à une source de liquide (2), un segment de pompe (9) pour le pompage du liquide jusqu'à un appareil d'injection (4) de liquide, et un conduit de drainage (15) connectable à la capsule (5), ledit conduit mentionné en premier (3), la capsule (5) et ledit conduit de drainage (15) étant prévus pour être connectés en série, caractérisé en ce qu'un capteur de pression (19), connectable au conduit (3) entre la pompe (1) et la capsule (5), est prévu pour commander une vanne de drainage (16) disposée dans le conduit de drainage (15) afin de régler la pression dans la capsule (5).

2. Système suivant la revendication 1, caractérisé en ce que ledit dispositif à tube souple comprend un conduit de dérivation (18) raccordé à l'aval du segment de pompe (9), ce conduit de dérivation étant connectable au capteur de pression (19) par l'intermédiaire d'un filtre (20) étanche au liquide mais perméable au gaz, ou d'une membrane élastique étanche.

3. Système suivant la revendication 2, caractérisé en ce que ledit conduit de dérivation (18) comporte en outre une soupape d'évent (21) qui comprend de préférence une membrane à auto-fermeture (23) qui peut être pénétrée par une canule (22).

4. Système suivant l'une quelconque des revendications 1 à 3, caractérisé en ce que le capteur de pression (19) est prévu pour ouvrir la vanne (16) dans le conduit de drainage (15) lorsqu'une certaine pression, de préférence réglable, est atteinte dans la capsule (5).

5. Système suivant l'une quelconque des revendications 1 à 4, caractérisé en ce que le capteur de pression (19) est prévu pour arrêter une nouvelle augmentation de pression, par réglage de la pompe (1) ou arrêt complet de la pompe lorsqu'une certaine pression maximale, de préférence réglable, est atteinte dans la capsule (5).

6. Système suivant l'une quelconque des revendications 1 à 3, caractérisé en ce que la vanne (16) du conduit de drainage (15) peut également être commandée manuellement.

7. Système suivant les revendications 4 et 5, caractérisé en ce qu'il comprend des moyens pour la commande commune desdites deux pressions réglables, de sorte que la vanne (16) du conduit de drainage (15) s'ouvre toujours lorsque la pression dans la capsule (5) atteint une certaine fraction de ladite pression maximale.

*Fig. 1*

0 185 658

Fig. 2

Q (ml/min)

Fig. 3

P (N/m²)